(19) **Europäisches Patentamt**

**European Patent Office**

**Office européen des brevets**

(11) **EP 1 205 703 B1**

(12) **EUROPÄISCHE PATENTSCHRIFT**

| | |
|---|---|
| (45) Veröffentlichungstag und Bekanntmachung des Hinweises auf die Patenterteilung:<br>**14.06.2006 Patentblatt 2006/24** | (51) Int Cl.:<br>**F16M 11/04** *(2006.01)* **G02B 7/00** *(2006.01)* |

(21) Anmeldenummer: **01126728.3**

(22) Anmeldetag: **09.11.2001**

(54) **Stativ, insbesondere für Operationsmikroskope**

Stand, in particular for surgical microscope

Support,en particulier pour microscope chirurgical

(84) Benannte Vertragsstaaten:
**DE FR GB**

(30) Priorität: **12.11.2000 DE 20019109 U**

(43) Veröffentlichungstag der Anmeldung:
**15.05.2002 Patentblatt 2002/20**

(73) Patentinhaber: **Leica Microsystems (Schweiz) AG**
**9435 Heerbrugg (CH)**

(72) Erfinder: **Metelski, Andrzej**
**8590 Romanshorn (CH)**

(74) Vertreter: **Rosenich, Paul**
**Patentbüro Paul Rosenich AG**
**BGZ**
**9497 Triesenberg (LI)**

(56) Entgegenhaltungen:
**DE-U- 20 019 105      US-A- 5 173 802**
**US-A- 5 667 186      US-A- 6 070 839**
**US-A- 6 129 319**

EP 1 205 703 B1

**Beschreibung**

Die Erfindung betrifft ein Stativ, insbesondere für Operationsmikroskope entsprechend dem Oberbegriff des Anspruches 1. Operationsmikroskope müssen über einen vorgegebenen Bereich leicht schwenkbar sein und sollten die danach eingestellte Position beibehalten. Aus diesem Grund sind Ausgleichsgewichte vorgesehen, die das Gewicht des Mikroskops und seiner Zusatzeinrichtungen kompensieren. Am häufigsten werden die Ausgleichsgewichte balkenwaagenartig angeordnet. Besondere Ausbildungsformen solcher Balkenwaagenanordnungen sind beispielsweise der Aufbau "OHS™" der Anmelderin, bei dem über Parallelogrammträger Ausgleichsgewichte von oben nach unten verlegt sind, sodass der Gesamtschwerpunkt des Stativs im unteren Bereich des Stativaufbaus liegt. Der Prinzipaufbau des OHS™ ist in der internationalen Patentanmeldung WO 97 13 997 A1 symbolisch dargestellt.

Ein weiterer Aufbau der Anmelderin "MS 1" sieht als Ausgleichseinrichtung für die leichte Bedienbarkeit des Mikroskops bzw. Beweglichkeit desselben im Raum und für die diesbezügliche Kompensation von Gewichtsänderungen am Mikroskop durch das Hinzugeben oder Wegnehmen von Zusatzeinrichtungen am Mikroskop erfolgt beim MS 1 über eine Druckfeder, die schräg bzw. diagonal in einem Parallelogrammträger eingespannt ist. Dieser Parallelogrammträger dient als schwenkbarer horizontaler Träger für das Mikroskop beim MS 1. In der WO 99 01 693 A1 ist der Aufbau des MS 1 symbolisch dargestellt. Die besondere Parallelogrammträgerkonstruktion wurde durch die Anmelderin in der europäischen Patentanmeldung EP 433 426 A1 veröffentlicht.

Zur verbesserten Kippsicherheit ist beim MS1 ein Schaltkasten vorgesehen, der sowohl die elektrische Stromversorgung für das Mikroskop, als auch seine Beleuchtungseinrichtung, seine Steuerungen o.dgl. und ggf. ein Zusatzgewicht enthält. Der Schaltkasten ist starr an der vertikalen Tragsäule des Stativs montiert und übernimmt dort lediglich einen Gewichtsausgleich über die vertikale Achse der senkrechten Tragsäule im Hinblick auf eine Verbesserung des Kippmomentes des Stativs.

In der DE 197 42 050 A1 wird Bezug genommen auf einen Aufsatz "Gewichtsausgleich an feinmechanischen Geräten" von H. Hilpert in Heft 2/1965 der Zeitschrift Feingerätetechnik, 14. Jahrgang.

In diesem Artikel aus dem Jahr 1965 wird auf verschiedene gewichtskompensatorische Massnahmen in der Feingerätetechnik eingegangen, die in erster Linie nicht durch ein Gegengewicht, sondern durch federkompensatorische Massnahmen (wie vergleichsweise auch bei der Konstruktion des MS 1) erzielt werden.

Die DD 221 571 A1 zeigt einen Stativaufbau mit einem Hebelarm, der durch eine Feder, die über einen Seilzug mit dem Hebelarm verbunden ist, Gewicht kompensiert. Am distalen Ende des Hebelarms befindet sich das Operationsmikroskop. Die Grundjustierung dieses Operationsmikroskops erfolgt über eine Gewindespindel, mit der das gehäusefeste Ende der Feder weiter vom Hebelarm weggezogen oder näher zu ihm hingeführt wird. Gewichtsänderungen am Mikroskop werden dadurch kompensiert, dass der Anlenkpunkt des Seilzugs relativ zum Hebelarm über eine Spindel verstellt wird.

Um in allen möglichen Winkellagen ein gleichmässiges Gegenmoment zu erzielen, ist es erforderlich, dass sich der erwähnte Angriffspunkt des Seilzugs auf einer Verbindungslinie zwischen der Drehachse des Hebelarms und dem Masseschwerpunkt des Mikroskops befindet. Dies wird durch das Betätigen einer Verstelleinrichtung in Form einer Schnecke erreicht, die eine mit dem Hebelarm verbundene Scheibe um die Drehachse des Hebelarms dreht.

Bei diesem Aufbau sind somit eine Fülle von Verstellmassnahmen erforderlich, um den gewünschten Effekt zu erzielen. Dabei wird durch die Scheiben-Schnecken-Konstruktion verhindert, dass - unabhängig vom Gewicht - eine beliebige Lage des Mikroskops gewählt werden kann. Abgesehen davon, zwingt die Konstruktion dieses bekannten Aufbaus zu einem hohen Gesamtschwerpunkt des Stativs, da sämtliche Gewichtsausgleichsvorrichtungen oberhalb des Mikroskops angeordnet sind.

Die DE 37 39 080 A1 gibt ebenso eine Federvorrichtung zum Gewichtsausgleich für Stative an, bei der Seilzüge mit Federn kombiniert zu einem Gewichtsausgleich führen sollen. Dabei geht es jedoch um die Kraftunterstützung einer Verstellbewegung, die eine Bedienperson an einem Handgriff ausübt. Es gibt von diesem Stand der Technik aus keine Lehre, eine Last in einem austarierten, "schwebenden Zustand" zu halten, wie dies bei Operationsmikroskopen gewünscht ist.

Demgegenüber ist in der US 5 397 323 ein Operationsroboter mit Para-Ilelogrammträgern vorgestellt, bei dem unter anderem das Gewicht des Instruments über einen Seilzug mittels Gegengewicht gewichtskompensiert gehalten wird. Der Seilzug ist dabei geschlossen aufgebaut, d.h. über eine obere und untere Umlenkrolle wird je ein Seil vom Instrument bis zum Gegengewicht geführt (Fig.3 der US 5397323).

Ein solcher Aufbau setzt voraus, dass das Gegengewicht in unmittelbarer Nähe des Instruments angebracht ist. Er wäre daher für den Einsatz an einem Operationsmikroskop nur schlecht anwendbar. Seine technische Lehre ist somit für die Adaption an ein Stativ für Mikroskope nicht nahegelegt.

Stative ohne Schwenkarm jedoch mit einem Gewichtsausgleich mittels Bandzügen über eine einzige Umlenkrolle unmittelbar an der Ständersäule wurden unter der Bezeichnung "Standard" und "Universal" auf den Markt gebracht. Diese verfügten jedoch über keinen Schwenkarm und es beschränkte sich der Gewichtsausgleich auf einen Ausgleich der Vertikallast des Tragarmes unmittelbar an der Ständersäule. Die Bänder, die die Kraft übertrugen, verliefen ausschliesslich parallel zur und unmittelbar neben der Ständersäule. Sie griffen nicht am Tragarm sondern an einem Ring unmittelbar an der Ständersäule an, der den seitlich abragenden Tragarm hielt. Bei einem Lastwechsel mussten auch am Ausgleichsgewicht Änderungen vorgenommen werden, wollte man einen Gewichtsausgleich erreichen.

Die DE 197 42 050 A1 offenbart einen Stativaufbau mit einem schwenkbaren Parallelogrammträger, der über einen Seilzug und eine Gewichtsausgleichsfeder so gewichtskompensiert ist, dass die zusätzlich vorhandenen Ausgleichsgewichte, die nach dem oben erwähnten Waagenprinzip wirken, besonders klein ausgebildet sein können. Bei diesem Aufbau wird der Seilzug in besonderer Form geführt, um den durch den endlichen Umlenkradius bedingten Gewichtsausgleichsfehler in einem weiten Schwenkbereich des Schwenkarms zu minimieren. Der Gewichtsausgleichsfehler wird durch diese Massnahme jedoch nicht eliminiert, sodass bei bestimmten Schwenkpositionen nach wie vor das Verstellen der Ausgleichsgewichte erforderlich ist.

Die US 6 070 839 offenbart einen weiteren Aufbau mit einem Schwenkarm und einer Seilzug-Feder-Konstruktion, die einen reinen Gewichtsausgleich - im Sinne des oben erwähnten Gewichtsausgleichs mit diagonaler Stützfeder - ermöglicht, ohne jedoch auch Ausgleichsmomente zu einer Verbesserung der Kippsicherheit beizutragen. Im Falle von Gewichtsänderungen wird der Anlenkpunkt des Seilzugs, vergleichbar dem Aufbau in der erwähnten DD 221571, über eine Spindel verschoben.

Die US 5 253 832 beschreibt ein Stativ mit einer zentral angeordneten Zugfeder für den Gewichtsausgleich. Dieser Aufbau bietet keine einfache Verstellmöglichkeit für geänderte Lasten, sodass Änderungen an einem Mikroskop oder an seinem Zubehör nicht ohne weiteres zu einer Justierung der Ausgleichskräfte führen können. Zudem ist es nachteilig, dass die Zugfeder - in Abhängigkeit von der Schwenkstellung des Tragarms - über einen unterschiedlichen Kompressions- bzw. Expansionsgrad verfügt, was aufgrund der Federkennlinie zu unterschiedlichen Ausgleichskräften und damit zu einem Ausgleichsverhalten führt, das über den Schwenkbereich des Tragarms unterschiedlich und somit für einen Anwender im chirurgischen Bereich unbrauchbar ist.

Gegenüber diesen bekannten Aufbauten liegt der Erfindung die Aufgabe zugrunde, ein neuartiges Stativ, insbesondere für Operationsmikroskope, zu schaffen, das mit möglichst geringem Verstellaufwand eine optimale Kompensationsmöglichkeit von Änderungen an der Last (Operationsmikroskop) bewirkt, unabhängig von der (Schwenk-) Lage des Mikroskops.

Gelöst wird diese Aufgabe durch die Merkmale des Anspruchs 1; insbesondere durch den Einsatz eines neuartigen Tariergetriebes, das einen veränderten Gewichtszustand an der Last (Operationsmikroskop) dadurch kompensiert, dass eine konstant angreifende Ausgleichskraft (Feder, Gewicht o.dgl.) in einem unterschiedlichen Übersetzungsverhältnis auf den Tragarm der Last einwirkt und zwar unabhängig von der Lage (Schwenklage) der Last.

Im Rahmen dieses grundsätzlichen Erfindungsgedankens liegen verschiedene Ausführungsformen und Weiterbildungen dieser Ausführungsformen, wie sie in den abhängigen Ansprüchen angegeben sind und sich aufgrund der Angaben, ggf. unter Beachtung der Lehren der unten erwähnten Patentanmeldungen vom selben Tag, dem Fachmann erschliessen.

Für besonders leistungsstarke Gewichtskompensation auf kleinem Raum sind Federn ideal. Sowohl Zug-, als auch Druckfedern eignen sich je nach Anbringunsort. Theoretisch wären Federn ideal, die über einen bestimmten Kompresssions- oder Expansionsweg die gleiche Kraft aufbringen. Solche Federn sind jedoch bei einem vergleichbaren Aufbau mit den vergleichbaren Parametern nicht einsetzbar. Daher werden vorzugsweise herkömmliche Federn benutzt, die jedoch mittels Kompensationsausgleich in Form von Kurven o.dgl. eine Bewegung (Hoch-Tief-Schwenken) der Last tolerieren, um stets gleiche Kompensationskraft bzw. Kompensationsmomente aufzubringen.

Als andere vorteilhafte Variante hat sich jener Aufbau herausgestellt, bei dem die Einrichtung für das Erzeugen einer konstanten Kraft nicht eine Feder, sondern ein Ausgleichsgewicht ist, das an einem Seilzug geführt ist.

Der Tragarm ist auch als Parallelogrammträger ausgebildet und der Arm des Tariergetriebes ist starr mit dem tragenden, gegebenenfalls mit dem oberen Träger des Parallelogrammträgers (2) verbunden.

In einer weiteren Ausgestaltung der Erfindung kann die Ausgleichskraftvorrichtung mit folgenden Bauelemente ausgestattet werden: Federn und/oder Hängegewichte, die über Seilrollen mit dem Verbindungsarm verbunden sind oder/und elektromotorische, hydraulische oder pneumatische Kraftaufbringer.

Es ist außerdem vorgesehen, dass der mit dem Tragarm starr verbundene Arm wenigstens annähernd rechtwinklig von diesem abragt.

In einer weiteren Ausgestaltung der Erfindung ist der Arm (11) als Parallelogrammträger ausgebildet ist, dessen beide Längsarme am gleichen Grundkörper (12) schwenkbar befestigt sind, an dem auch der Tragarm (2) schwenkbar befestigt ist, wobei das Verbindungselement als Verbindungsarm (10) ausgebildet und mit dem anderen Ende der beiden Arme (11) gelenkig verbunden ist.

Das Lastausgleichseinheit (18) und/oder das Tariergetriebe (6) kann wenigstens teilweise auf der einen Seite einer vertikalen Stützachse (1) liegen, während der Tragarm (2) sich auf der anderen Seite befindet, wobei die vertikale Stützachse (1) jene Achse ist, um die das Stativ hinsichtlich Kippsicherheit untersucht wird.

Der Sensor kann als ein Scherkraftsensor ausgebildet sein, der am nicht tragenden Arm des Parallelogrammträgers an einer verjüngten Stelle desselben angeordnet ist.

In einer weiteren Ausgestaltung der Erfindung ist an einer der Lagerstellen des Tragarmes (2) oder des Tariergetriebes (6; 106) oder des Wagens (7) oder des Armes (11) eine Bremse zum Einbremsen des Stativs in einer gewählten Lage angeordnet ist, wobei die Bremse elektrisch oder elektromotorisch oder elektromagnetisch ansteuerbar ist.

Das beanspruchte Verfahren zeichnet sich dadurch aus, dass der Angriffspunkt der konstanten Kraft bei aktivierter Bremse zunächst laufend verändert wird und diese Laständerungen fortlaufend von einem Sensor registriert werden. Über eine Schaltung wird dabei das Sensorsignal "Gleichgewicht" (der Sensor gibt beispielsweise kein Signal mehr ab) registriert. In immer kleiner werdenden Schritten wird dann der Angriffspunkt der konstanten Kraft um diesen Gleichgewichtspunkt herum so lange verändert, bis ein Gleichgewichtszustand bzw. eine Kompensierung des Gewichts am Lastarm vorliegt.

Im Rahmen der vorliegenden Anmeldung ergeben sich bei einer besonderen Ausgestaltung des Tariergetriebes auch kippmomentunabhängige Effekte, wenn das Tariergetriebe so aufgebaut ist, dass wenigstens ein Teil seiner Mechanik auf der der Last abgewandten Seite des Vertikalträgers angebracht ist und dass wenigstens ein Teil der die konstante Kraft aufbringenden Einrichtung auf derselben Seite liegt.

Anhand von Skizzen wird die Erfindung beispielhaft näher erläutert. Es zeigen dabei:

Fig.1 -    Den prinzipiellen Aufbau einer erfindungsgemäßen Ausgleichsvorrichtung mit einer federgestützten Kraftkompensation;

Fig.2 -    Das Prinzip des Tariergetriebes;

Fig.3 -    Zwei Diagramme, die die Wirkungsweise der Federeinheit gemäss Fig.1 zur Erzeugung einer konstanten Kraft darstellen;

Fig.4 -    Den Wirkmechanismus des erfindungsgemäßen Tariergetriebes bedingt durch die Kurve bei unterschiedlichen Schwenklagen des Stativs;

Fig.5 -    Ein Detail aus Fig.1;

Fig.6 -    Einen möglichen konstruktiven Aufbau des Tariergetriebes.

Die Figuren werden übergreifend beschrieben, gleiche Bezugszeichen bedeuten gleiche Bauteile, Bezugszeichen mit gleichen Nummern, jedoch unterschiedlichen Indizes bedeuten geringfügig unterschiedliche Bauteile mit gleichen Aufgaben beziehungsweise ähnlichen Wirkungen.

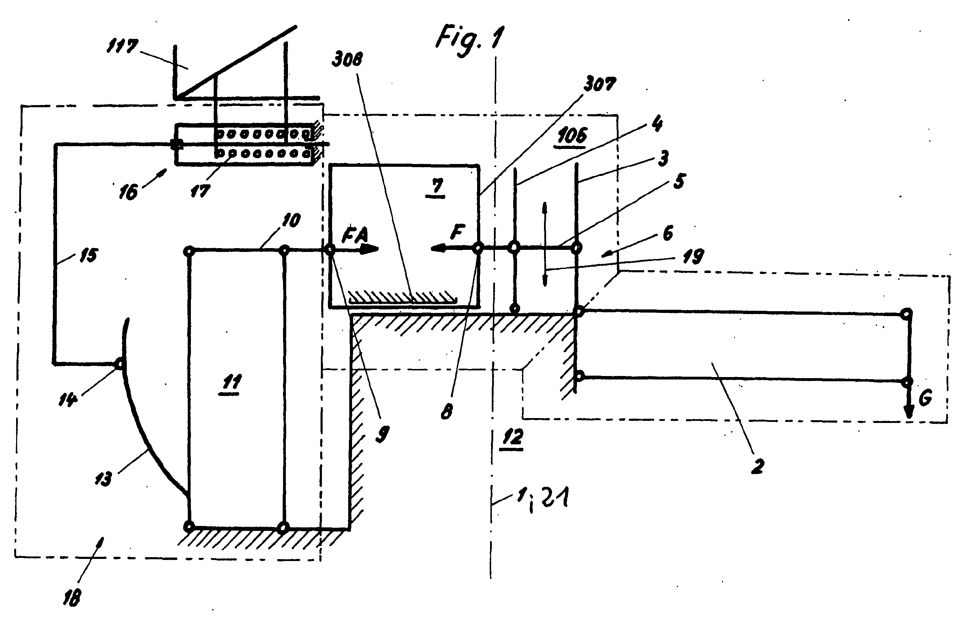

Fig1: Mit 1 ist die vertikale Achse des vertikalen Ständers des Stativs und/oder jene Achse angegeben, um die das Stativ nach seiner Kippsicherheit untersucht wird. Das könnte z.B. eine auf den Boden vertikale Achse durch eines der Stützräder des Stativs sein. Es handelt sich dabei somit in der Regel um eine virtuelle Achse. Diese Achse fällt beispielsweise mit einer Rohrachse eines Ständerrohres 21 zusammen. Sie ist dann z.B. als virtuelle Achse zu verstehen, wenn z.B. dieses Ständerrohr 21 selbst schwenkbar ist (wie beispielsweise beim OHS™) oder weil der Stativaufbau auch gänzlich ohne vertikales Ständerrohr 21 auskommen könnte (z.B. bei einem wandgestützten Stativ).

Mit G ist die Last, bzw. das Operationsmikroskop angegeben, das am distalen Ende eines schwenkbaren Parallelogrammträgers 2 gehalten ist. Der obere Arm des Parallelogrammträgers 2 ist mit einem Arm 3 starr verbunden, der mit einem weiteren parallelen Arm 4 und einem Verbindungsarm 5 zu einem weiteren Parallelogramm 6 verbunden ist. Dieses Parallelogramm 6 bildet das Tariergetriebe 106, in dem der Verbindungsarm 5 in seiner parallelen Lage höhenverstellbar ist, wodurch die Geometrie des Parallelogramms veränderbar ist (Pfeil 19). Ein Verschwenken des Parallelogrammträgers 2 nach oben oder nach unten führt zu einem Verschwenken des Parallelogramms 6 nach links oder nach rechts. Aus Figur 4 ist die äußerste Lage des Verbindungsarms 5 ersichtlich, bei der die größte Hebelarmübersetzung besteht, d.h. bei der G die größte Last annehmen kann. Aus Figur 4 sieht man außerdem, wie sich beim Verschwenkvorgang des Parallelogrammträgers 2 die Geometrie des Parallelogramms 6 verändert, woraus sich ein automatischer Kompensationseffekt bei den Ausgleichskräften ergibt.

Dies nach der Formel M(G)=M(F) bzw. l1x G = h1 x F bzw. l2 x G = h2 x F, wobei M(G) das Moment der Last und M(F) das Moment der Gegenkraft angibt.

$$ l2 = l1 \times \cos \alpha \qquad h2 = h1 \times \cos \alpha \qquad \frac{l2}{h2} = \frac{l1.\cos\alpha}{h1.\cos\alpha} = \frac{l1}{h1} \ . $$

Im Falle einer Gewichtsänderung an G muss man somit erfindungsgemäss lediglich den Verbindungsarm 5 parallel entlang dem Arm 3 verschieben, um dementsprechend h1 zu ändern, damit bei konstanter Ausgleichskraft FA die Kompensation der Gewichtsänderung erzielt wird.

Ausgehend von dem Tariergetriebe 6,106 (Parallelogramm) können nun im Rahmen der Erfindung die verschiedensten Massnahmen zur Erzielung der konstanten Ausgleichskraft FA angewendet werden. Das können einerseits Hängegewichte sein, die über Seilrollen mit dem Verbindungsarm 5 verbunden sind, das können aber auch andere Formen herkömmlicher Kraftaufbringung sein, wie beispielsweise Federn (vgl. z.B. den Aufbau gem. US-5253832 mit seiner Zugfeder im vertikalen Ständer, elektromotorische, hydraulische oder pneumatische Kraftaufbringer o.dgl.

Bei dem vorliegenden Ausführungsbeispiel nach Fig.1 ist ein Wagen 7 oder ein Schlitten vorgesehen, der einerseits eine Rolle 8 trägt, die mit dem Verbindungsarm 5 starr verbunden ist und andererseits eine Rolle 9 trägt, die mit einem Verbindungsarm 10 eines weiteren Parallelogrammträgers 11 verbunden ist. Der Parallelogrammträger 11 ist wie der Parallelogrammträger 2 und das Parallelogramm 6 an einem gemeinsamen Tragbauteil 12 angelenkt. Der Parallelogrammträger 11 verfügt über eine Steuerkurve 13, an der mindestens eine Rolle 14 gleitet, die mit einem Verbindungsarm 15 auf einem Kraftspeicher 16 aufgestützt ist.

Der Kraftspeicher 16 kann verschiedenste Ausbildungsformen aufweisen und schränkt die Erfindung nicht ein. Im vorliegenden Fall ist symbolisch eine Druckfeder 17 angegeben, die über den Verbindungsarm 15 und die Rolle 14 einen Zug auf den Parallelogrammträger 11 nach links ausübt. Dieser Zug wird als Kraft FA von der Rolle 9 auf den Wagen 7 und durch diesen über die Rolle 8 auf den Verbindungsarm 5 übertragen, wodurch die Last G kompensiert ist.

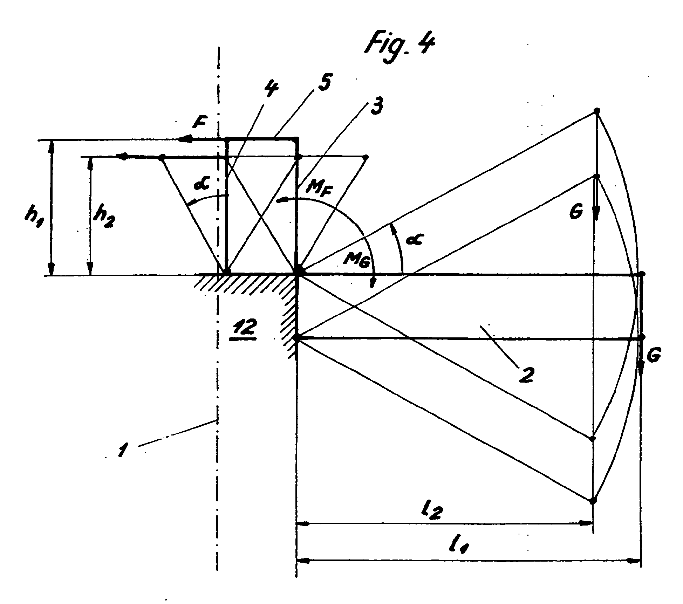

Die besondere Ausbildung der Steuerkurve 13 ermöglicht es, dass die Kraft FA stets konstant ist. Dies erfolgt durch folgenden Zusammenhang: Bei einem Hochschwenken von G bzw. vom Parallelträger 2 wird der Verbindungsarm 5 nach hinten (links in der Zeichnungsebene) verschoben. Dies führt zu einem Verschieben des Wagens 7 und des Verbindungsarms 10 nach hinten. Dadurch schwenkt das Parallelogramm 11 nach hinten und gibt somit der Druckfeder 17 nach, die sich entspannt. Aufgrund der Federeigenschaft würde dies ohne Kurve 13 zu einer Abnahme der Ausgleichskraft FA führen und damit zu einer Reduktion der Gewichtskompensation (Fig.3). Aufgrund der Ausbildung von der Kurve 13 wird dieser Effekt jedoch ausgeglichen (vgl. Fig.3), so dass unabhängig von der Schwenklage der Last G eine konstante Ausgleichskraft FA wirkt. Der rechtsschraffierte Bereich in Fig.3 zeigt den nichtkompensierten Bereich bei hmin und der linksschraffierte Bereich zeigt den nichtkompensierten Bereich bei hmax. Bei dem Aufbau der Federeinheit 18 handelt es sich somit um eine Vorrichtung zur Aufbringung einer konstanten Kraft. Wie schon erwähnt, kann diese Vorrichtung durch andere Vorrichtungen zur Aufbringung konstanter Kräfte ersetzt werden, um gemeinsam mit dem erfindungsgemässen Tariergetriebe ein erfindungsgemässes Austarieren der Last G zu ermöglichen.

Symbolisch ist oberhalb der Feder 17 ein Diagramm 117 angegeben, das dem aus Fig.3 entspricht.

Bei bestimmten Ausführungsformen kann, - zählt man den Wagen 7 noch zum Tariergetriebe 106 - ein Teil des Tariergetriebes links der Achse 1 liegen, während die Federeinheit 18 vollständig auf der linken Seite liegt. Das Eigengewicht des Wagens 7 und der Federeinheit 18 ist bei diesen Ausführungsformen somit um die Achse 1 verteilt, sodass die Kippsicherheit über der Achse 1 nicht nachteilig beeinflusst wird.

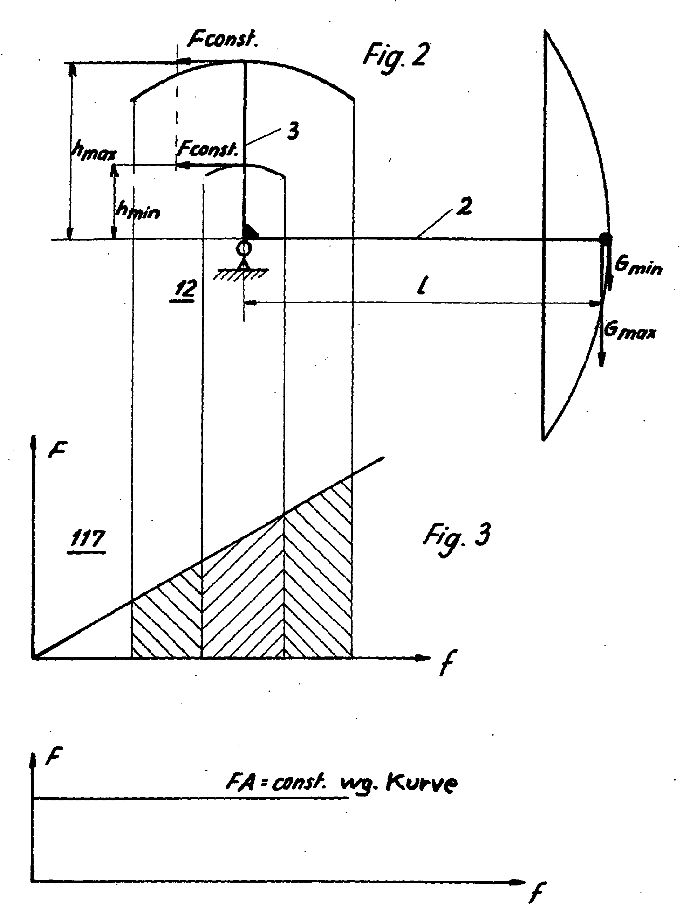

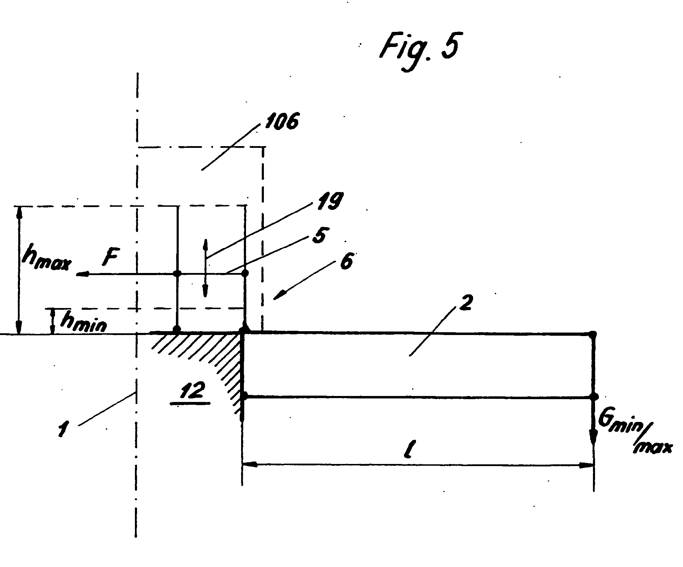

In den Fig.2 und 5 ist die Auswirkung des Tariergetriebes verständlich dargestellt.

G max x l = F x h max; G min x l = F x h min, wobei wie schon erwähnt, F konstant ist.

Mit einem solchen erfindungsgemäßen Aufbau kann die Last G beispielsweise zwischen G max = 30 kp und G min = 5 kp betragen. In den dargestellten Zeichnungen sind jeweils Parallelogrammträger bzw. Parallelogramme angegeben, weil diese hinsichtlich Biegeverhalten und Schwingungen für den Stativbau günstig einzusetzen sind. Die Erfindung ist jedoch nicht auf Parallelogrammträger eingeschränkt, sondern könnte vielmehr auch mit normalen Biegeträgern funktionieren. Das gilt sowohl für den Tragarm 2, an dem die Last G hängt, als auch für den Parallelogrammträger 11 und das Parallelogramm 6, wobei bei letzterem aus Parallelführungsgründen für den Verbindungsarm 5 ein Parallelogramm in jedem Fall bevorzugt ist.

Im Rahmen der Erfindung ist es nicht wesentlich, ob das Tariergetriebe 106 oberhalb - wie dargestellt - oder unterhalb des Parallelogrammträgers 2 angeordnet ist. Bei einer Anordnung unterhalb ist dementsprechend die Federeinheit 18 spiegelbildlich auszuführen, indem auch die Druckfeder 17 zu einer Zugfeder umgewandelt werden müsste.

Das obere Diagramm der Fig.3 gibt beispielhaft etwa den Verlauf der Federkraft bei verändertem Weg (Komprimierung der Feder) an. Dies entspricht etwa einer normalen Druckfeder. Um diesen Verlauf in seiner Wirkung abzuändern, damit am Wirkpunkt der Federkraft, nämlich am Wagen 7 bzw. am Verbindungsarm 5 die Kraft konstant ist, wie im unteren Diagramm der Fig.3 gezeigt, ist die Kurve 13 vorgesehen. Sie bewirkt, dass bei einer Verschwenkstellung der Last G und einer damit verbundenen Verschwenkstellung des Tariergetriebes 106 und einer damit verbundenen Verschwenkstellung des Parallelogrammträgers 11 und einer damit verbundenen Komprimierung oder Dekomprimierung der Feder 17 die auf den Verbindungsarm 10 bzw. den Wagen 7 ausgeübte Zugkraft (FA) konstant bleibt.

Einher mit diesem mechanischen Aufbau geht der Effekt, dass bei gleichem Winkelweg des Tragarmes 2 der Wagen 7 unterschiedliche Wege fährt, in Abhängigkeit von der Last G, sofern diese durch Einstellung des Tariergetriebes 106 kompensiert ist.

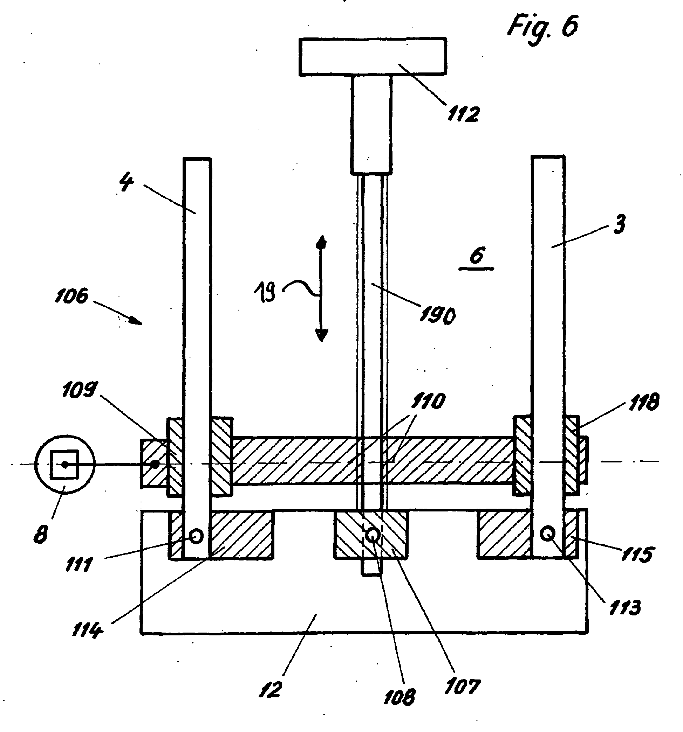

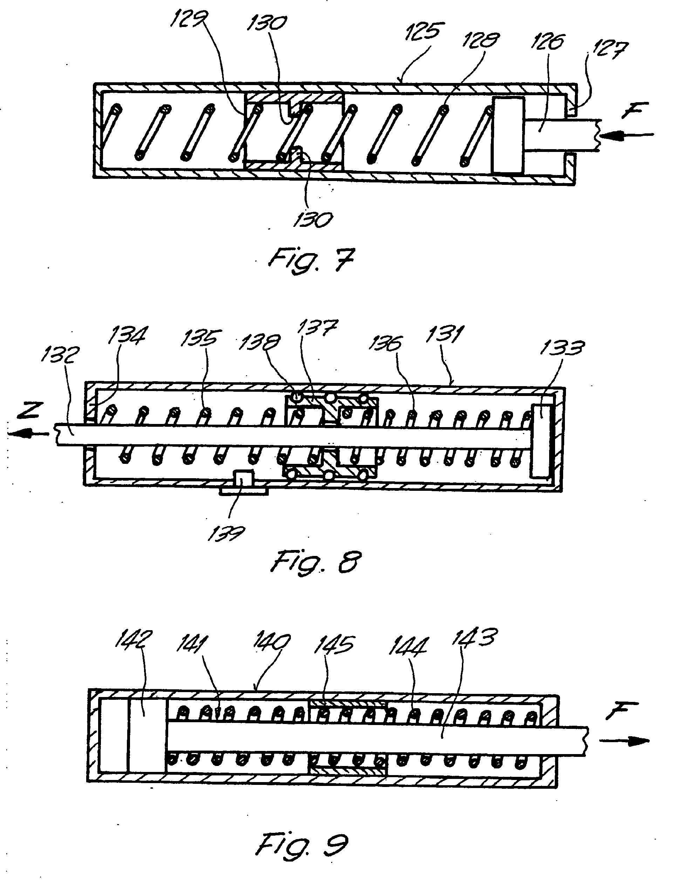

Der ausgeführte Aufbau nach Fig. 6 umfasst die Parallelogrammarme 3 und 4, die über Schwenkhalterungen 115 bzw. 114 am Grundkörper 12 schwenkbar gelagert sind. Sie tragen Parallelführungen 109 bzw. 118 für den Verbindungsarm 5, der seinerseits eine Gewindebohrung 110 für die Verstellspindel 190 aufweist. Diese trägt an ihrem oberen Ende einen Handgriff 112, könnte jedoch, wie schon erwähnt, auch motorisch antreibbar sein. Die Spindel 190 ist an ihrem unteren Ende über ein Schwenklager 107 am Grundkörper 12 gelagert. Justierspindeln dienen der parallelen Einjustierung des Verbindungsarmes 5 in Bezug auf den Grundkörper 12. Unter Trägern im Sinne der Patentansprüche sind sowohl einzelne Tragarme als auch Parallelogrammträger oder ähnliche Konstruktionen zu verstehen.

Bezugszeichenliste

| | |
|---|---|
| 1 - | Vertikale Achse des vertikalen Ständers |
| 2 - | Tragarm bzw. Parallelogrammträger |
| 3 - | Arm |
| 4 - | Arm |
| 5 - | Verbindungsarm |
| 6 - | Parallelogrammträger bzw. 106 -Tariergetriebe |
| 7 - | Transferelement, vorzugsweise Wagen bzw. Schlitten |
| 8 - | Rolle |
| 9 - | Rolle |
| 10 - | Verbindungsarm |
| 11 - | Tragarm bzw. Parallelogrammträger |
| 12 - | Tragbauteil bzw. Grundkörper |
| 13 - | Steuerkurve |
| 14 - | Rolle |
| 15 - | Verbindungsarm |
| 16 - | Kraftspeicher |
| 17 - | Druckfeder |
| 18 - | Federeinheit bzw. Lastausgleichseinheit |
| 19 - | Pfeil |
| 20 - | Stativfuss |
| 21 - | Ständerrohr |
| 106 - | Tariergetriebe (vgl. 6) |
| 107 - | Schwenklager |
| 108 - | Schwenkachsen für Schwenklager |
| 109 - | Parallelführungen |
| 110 - | Gewindebohrung |
| 111 - | Schwenkachsen für Schwenklager |
| 113 - | Schwenkachsen für Schwenklager |
| 114 - | Schwenklager |
| 115 - | Schwenklager |
| 112 - | Handgriff |
| 117 - | Kraft-Weg-Diagramm der Feder 17 |
| 118 - | Parallelführungen |
| 190 - | Verstellspindel |
| 307 - | Wange des Wagens 7 |
| 308 - | Führung für Wagen |
| G - | Last bzw. Gewicht des Mikroskops |
| AG - | Ausgleichsgewicht |
| F - | Kraft |
| FA - | Konstante Ausgleichskraft |

**Patentansprüche**

**1.** Stativ, insbesondere für Operationsmikroskope, mit einer vertikalen Achse (1), mit einem Tragarm (2) mit einer Länge l, für eine Last G, die lastseitig der vertikalen Achse (1) mit einer Kraft F. wirkt und einem Ausgleichsarm mit wenigstens einer Lastausgleichseinheit (18), die mit einer Lastausgleichskraft FA ausgleichseitig der vertikalen Achse (1) der Kraft F entgegenwirkt, **dadurch gekennzeichnet, dass** die Lastausgleichseinheit **(18)** so aufgebaut ist, dass sie unabhängig von der Last G mit konstanter Lastausgleichskraft FA gegen die Kraft F wirkt und dass am Tragarm (2) ein Tariergetriebe (6; 106) angeordnet ist, das die Angriffspunkte der Kraft F und der konstanten Lastausgleichskraft FA am Tragarm (2) oder an einem mit dem Tragarm (2) starr verbundenen Arm (3) in einer Höhe parallel zu der vertikalen Achse (1) so verändert, dass das Produkt aus Kraft F x Weg h der Veränderung des Angriffspunktes der Kraft F am Tragarm (2) oder am Arm (3) dem Produkt aus veränderter Last G x Länge I des Tragarms (2) entspricht.

2. Stativ nach Anspruch **1, dadurch gekennzeichnet, dass** zwischen dem Tariergetriebe (6; 106) und der Lastausgleichseinheit **(18)** ein Transferelement (7), vorzugsweise ein Wagen vorgesehen ist, an dem einerseits ein kraftübertragendes Übertragungselement **(5)** des Tariergetriebes (6; 106) und andererseits die Lastausgleichseinheit **(18)** angreift, derart, **dass** das Übertragungselement entlang des Transferelements **(7)** gleiten kann und wobei das Transferelement (7) in Kraftrichtung verschiebbar ist.

3. Stativ nach Anspruch 2, **dadurch gekennzeichnet, dass** das Übertragungselement (5) über wenigstens eine Spindel (190), die gegenüber dem Grundkörper (12) abgestützt ist und entlang des Tragarms (2) oder entlang des mit diesem starr verbundenen Arms (3) verstellbar ist, wobei die Spindel (190) verstellbar ausgebildet ist und die Verstellung der Spindel (190) rechnergesteuert erfolgt und am Tragarm (2) mittels Sensor die Laständerung der Last G ermittelt wird.

4. Stativ nach einem der vorhergehenden Ansprüche, **dadurch gekennzeichnet, dass** das Tariergetriebe (6; 106) aus einem Parallelogrammträger (2) aufgebaut ist, wobei einer der Arme des Parallelogrammträgers durch den starr verbundenen Arm (3) gebildet wird, und dieser einen dazu parallelen Arm (4) aufweist, wobei beide Arme (3 und 4) an einem Grundkörper (12) angelenkt sind, an dem auch der Tragarm (2) angelenkt ist und wobei der Verbindungsarm (5) an beiden Armen (3 und 4) beweglich gelagert und entlang der beiden Arme (3 und 4) parallel verschiebbar ist.

5. Stativ nach einem der Ansprüche 2-4, **dadurch gekennzeichnet, dass** das Übertragungselement als Verbindungsarm (5) ausgebildet ist und dass der Angriff der Lastausgleichseinheit (18) ebenso über einen Verbindungsarm (10) gebildet ist, wobei wenigstens einer der Verbindungsarme (5, 10) an seinen Angriffsstellen Rollen (8, 9) aufweist, die entlang den Wangen des Transferelements (7) rollen.

6. Stativ nach Anspruch 5, **dadurch gekennzeichnet, dass** die Lastausgleichseinheit (18) eine Druck- oder Zugfeder (17) umfasst, die über einen Verbindungsarm (15) auf eine Steuerkurve (13) wirkt, die mit einem Arm (11) starr verbunden ist, der über das Verbindungselement (10) die Lastausgleichskraft FA auf das Transferelement (7) überträgt und die Steuerkurve (13) so ausgebildet ist, dass trotz Verschwenken des Arms (11) nach links oder rechts eine konstante Lastausgleichskraft FA vom Verbindungsarm (10) abgreifbar ist.

7. Stativ nach Anspruch 2, **dadurch gekennzeichnet, dass** der Verbindungsarm (5) oder das Transferelement (7) mit einem Seilzug verbunden ist, der über eine Rolle nach unten umgelenkt ist, wobei am anderen Ende des Seilzugs ein Ausgleichsgewicht in seiner Höhenlage frei beweglich angebracht ist.

8. Stativ nach Anspruch 6, **dadurch gekennzeichnet, dass** zwischen der Feder (17) und einem von dieser Feder (17) beaufschlagten Parallelogramm (11) eine Kompensationskurve (13) vorgesehen ist, die beim Abgreifen der Kraft F an dem Verbindungsarm (10) des Parallelogramms (11) in Abhängigkeit von der Winkelstellung der Längsarme des Parallelogramms (11) das wirksame Moment konstant hält.

9. Stativ nach Anspruch 6 oder 8, **dadurch gekennzeichnet, dass** eine Vorrichtung zur Übertragung der Lastausgleichskraft FA zwischen der Feder (17) und einer linear bewegbaren Last G vorgesehen ist, wobei zwischen der Last G und der Feder (17) eine Kurvensteuerung (13) angeordnet ist, die den Weg der Last G so kompensiert, dass die Feder (17) nicht komprimiert oder entspannt wird.

10. Verfahren zum Kompensieren der Last G an einem Tragarm (2) eines Stativs nach mindestens einem der vorherigen Ansprüche, **dadurch gekennzeichnet, dass** der Angriffspunkt der konstanten Lastausgteichskraft FA so lange verändert wird, bis die Last G am Tragarm (2) kompensiert ist und dieser Angriffspunkt der konstanten Lastausgleichskraft FA zur genauen Einstellung des Gleichgewichts mehrmals hintereinander eingestellt wird.

**Claims**

1. Stand, in particular for surgical microscopes, having a vertical axis (1), a support arm (2) with a length I, for a load G which acts at the load end of the vertical axis (1) with a force F, and a compensating arm with at least one load-

7

equalizing unit (18) which, with a load equilibrant FA, counteracts the force F at the equalizing end of the vertical axis (1), **characterized in that** the load-equalizing unit (18) is constructed such that, independently of the load G, it acts with constant load equilibrant FA counter to the force F, and **in that** arranged on the support arm (2) there is a counterbalancing transmission (6; 106) which changes the points of engagement of the force F and of the constant load equilibrant FA on the support arm (2), or on an arm (3) rigidly connected to the support arm (2), in a height h parallel to the vertical axis (1) such that the product of force F x travel h of the change of the point of engagement of the force F on the support arm (2) or on the arm (3) corresponds to the product of changed load G x length l of the support arm (2).

2. Stand according to Claim 1, **characterized in that** provided between the counterbalancing transmission (6; 106) and the load-equalizing unit (18) is a transfer element (7), preferably a carriage on which, on the one hand, a force-transmitting transmission element (5) of the counterbalancing transmission (6; 106) acts and, on the other hand, the load-equalizing unit (18) acts in such a way that the transmission element can slide along the transfer element (7), it being possible for the transfer element (7) to be displaced in the direction of force.

3. Stand according to Claim 2, **characterized in that** the transmission element (5) can be adjusted via at least one spindle (190), which is supported with respect to the basic body (12), and along the support arm (2) or along the arm (3) rigidly connected to the latter, the spindle (190) being designed to be adjustable and the adjustment of the spindle (190) being performed under computer control and the change in the load G being determined at the support arm (2) by means of a sensor.

4. Stand according to one of the preceding claims, **characterized in that** the counterbalancing transmission (6; 106) is constructed from a parallelogram carrier (2), one of the arms of the parallelogram carrier being formed by the rigidly connected arm (3), and the latter having an arm (4) parallel thereto, both arms (3 and 4) being pivoted to a basic body (12) to which the support arm (2) is also pivoted and the connecting arm (5) being movably mounted on both arms (3 and 4) and being capable of parallel displacement along the two arms (3 and 4).

5. Stand according to one of Claims 2-4, **characterized in that** the transmission element is designed as a connecting arm (5) and **in that** the load-equalizing unit (18) likewise acts via a connecting arm (10), at least one of the connecting arms (5, 10) having at its points of action rollers (8, 9) which roll along the cheeks of the transfer element (7).

6. Stand according to Claim 5, **characterized in that** the load-equalizing unit (18) comprises a pressure or tension spring (17) which acts via a connecting arm (15) on a control cam (13) which is rigidly connected to an arm (11) which transmits the load equilibrant FA to the transfer element (7) via the connecting element (10) and the control cam (13) is designed such that despite pivoting of the arm (11) to the left or right it is possible to tap a constant load equilibrant FA from the connecting arm (10).

7. Stand according to Claim 2, **characterized in that** the connecting arm (5) or the transfer element (7) is connected to a cable pull which is deflected downwards over a roller, a balance weight being attached in a freely movable fashion as to its level at the other end of the cable pull.

8. Stand according to Claim 6, **characterized in that** provided between the spring (17) and a parallelogram (11) to which this spring (17) is applied is a compensation cam (13) which keeps constant the effective moment upon tapping of the force F at the connecting arm (10) of the parallelogram (11), as a function of the angular position of the longitudinal arms of the parallelogram (11).

9. Stand according to Claim 6 or 8, **characterized in that** there is provided an apparatus for transmitting the load equilibrant FA between the spring (17) and a linearly movable load G, where arranged between the load G and the spring (17) is a cam controller (13) which compensates the path of the load G such that the spring (17) is not compressed or untensioned.

10. Method for compensating the load G on a support arm (2) of a stand according to at least one of the preceding claims, **characterized in that** the point of engagement of the constant load equilibrant FA is changed until the load G on the support arm (2) is compensated, and this point of engagement of the constant load equilibrant FA is adjusted several times in succession in order to precisely adjust the balance weight.

**Revendications**

1. Statif, en particulier pour microscope opératoire, avec un axe vertical (1), avec un bras porteur (2) d'une longueur l, pour une charge G qui agit du côté charge de l'axe vertical (1) avec une force F, et un bras de compensation avec au moins une unité de compensation de charge (18) qui réagit contre la force F avec une force de compensation de charge FA du côté compensation de l'axe vertical (1), **caractérisé en ce que** l'unité de compensation de charge (18) est construite de telle sorte qu'elle agit indépendamment de la charge G avec une force de compensation de charge constante FA contre la force F et **en ce que** sur le bras porteur (2) un engrenage de tarage (6 ; 106) est disposé qui modifie les points d'attaque de la force F et de la force de compensation de charge constante FA sur le bras porteur (2) ou sur un bras (3) relié de façon rigide au bras porteur (2) à une hauteur h parallèle à l'axe vertical (1) de telle sorte que le produit de la force F x course h de la modification du point d'attaque de la force F sur le bras porteur (2) ou sur le bras (3) correspond au produit de la charge modifiée G x la longueur l du bras porteur (2).

2. Statif selon la revendication 1, **caractérisé en ce qu'**entre l'engrenage de tarage (6 ; 106) et l'unité de compensation de charge (18), un élément de transfert (7), de préférence un chariot, est prévu auquel s'appliquent d'une part un élément de transmission (5), transmettant une force, de l'engrenage de tarage (6 ; 106), et d'autre part l'unité de compensation de charge (18), de telle sorte que l'élément de transmission peut coulisser le long de l'élément de transfert (7), et l'élément de transfert (7) étant mobile dans la direction de la force.

3. Statif selon la revendication 2, **caractérisé en ce que** l'élément de transmission (5) est réglable par au moins une broche (190), qui est supportée face au corps de base (12) et mobile le long du bras porteur (2) ou le long du bras (3) relié de façon rigide à celui-ci, la broche (190) étant réalisée de façon réglable et le réglage de la broche (190) s'effectuant par commande informatique et la modification de charge de la charge G étant déterminée sur le bras porteur (2) au moyen d'un capteur.

4. Statif selon l'une quelconque des revendications précédentes, **caractérisé en ce que** l'engrenage de tarage (6 ; 106) se compose d'un support en parallélogramme (2), dans lequel l'un des bras du support en parallélogramme est formé par le bras (3) relié de façon rigide, et ce dernier présente un bras (4) parallèle à celui-ci, les deux bras (3 et 4) étant articulés sur un corps de base (12) auquel le bras porteur (2) est également articulé, et le bras de liaison (5) étant logé de façon mobile sur les deux bras (3 et 4) et mobile en parallèle le long des deux bras (3 et 4).

5. Statif selon l'une quelconque des revendications 2 à 4, **caractérisé en ce que** l'élément de transmission est réalisé comme un bras de liaison (5) et **en ce que** l'attaque de l'unité de compensation de charge (18) est également formée par l'intermédiaire d'un bras de liaison (10), dans lequel au moins l'un des bras de liaison (5, 10) présente des rouleaux (8, 9) au niveau de ses points d'attaque qui roulent le long des joues de l'élément de transfert (7).

6. Statif selon la revendication 5, **caractérisé en ce que** l'unité de compensation de charge (18) comprend un ressort de pression ou de traction (17) qui agit par l'intermédiaire d'un bras de liaison (15) sur une came de commande (13) qui est reliée de façon rigide à un bras (11) qui transmet par l'intermédiaire de l'élément de liaison (10) la force de compensation de charge FA à l'élément de transfert (7) et la came de commande (13) est réalisée de telle sorte que malgré le pivotement du bras (11) à gauche ou à droite une force de compensation de charge constante FA puisse être prélevée au niveau du bras de liaison (10).

7. Statif selon la revendication 2, **caractérisé en ce que** le bras de liaison (5) ou l'élément de transfert (7) est relié à un câble Bowden qui est renvoyé vers le bas par l'intermédiaire d'un rouleau, un contrepoids étant monté de façon librement mobile dans sa position de hauteur à l'autre extrémité du câble Bowden.

8. Statif selon la revendication 6, **caractérisé en ce qu'**entre le ressort (17) et un parallélogramme (11) sollicité par ce ressort (17), une came de compensation (13) est prévue qui maintient le moment effectif constant lorsque la force F sur le bras de liaison (10) du parallélogramme (11) est prélevée en fonction de la position angulaire des bras longitudinaux du parallélogramme (11).

9. Statif selon la revendication 6 ou 8, **caractérisé en ce qu'**un dispositif de transmission de la force de compensation de charge FA entre le ressort (17) et une charge mobile de façon linéaire G est prévu, dans lequel entre la charge G et le ressort (17) une came de commande (13) est disposée qui compense la course de la charge G de telle sorte que le ressort (17) n'est ni comprimé ni détendu.

10. Procédé de compensation de la charge G sur un bras porteur (2) d'un statif selon au moins l'une quelconque des revendications précédentes, **caractérisé en ce que** le point d'attaque de la force de compensation de charge constante FA reste modifiée jusqu'à ce que la charge G sur le bras porteur (2) ait été compensée et ce point d'attaque de la force de compensation de charge constante FA ait été réglé plusieurs fois de suite pour un réglage précis de l'équilibre.

Fig. 1

Fconst.

*Fig. 2*

3

Fconst.

$h_{max}$

$h_{min}$

12

2

$\ell$

$G_{min}$

$G_{max}$

F

117

*Fig. 3*

f

F

FA = const. wg. Kurve

f

Fig. 4

# Fig. 5

Fig. 6

Fig. 7

Fig. 8

Fig. 9